# EUROPÄISCHE PATENTSCHRIFT

(11) **EP 0 736 299 B1**
(45) Veröffentlichungstag und Bekanntmachung des Hinweises auf die Patenterteilung: **14.11.2001**
(21) Anmeldenummer: 96105334.5
(22) Anmeldetag: 03.04.1996
(51) Int. Cl.: A61K 9/127

(54) **Verfahren zur Herstellung einer liposomalen, in Wasser dispergierbaren, oral zu verabreichenden, festen, trockenen therapeutischen Formulierung**
Process for preparing a liposomal, water-dispersable, solid, dry, therapeutic formulation for oral administration
Procédé de préparation d'une formulation thérapeutique, solide et sèche de liposomes, dispersible dans l'eau pour administration orale

(30) Priorität: 03.04.1995 CH 92395
(43) Veröffentlichungstag der Anmeldung: 09.10.1996
(73) Patentinhaber: Cerbios-Pharma S.A., CH-6917 Barbengo (CH)
(72) Erfinder: Mazzei, Giovanni, 6515 Gudo (CH); Crivelli, Paolo, Dr., CH-6927 Agra (CH); Müller, Michele, Dr., 6918 Figino (CH); Marazza, Fabrizio, Dr., 6986 Novaggio (CH)
(74) Vertreter: Zink-Wild, Markus Peter

(56) Entgegenhaltungen:
- EP-A- 0 087 993
- EP-A- 0 170 642
- WO-A-88/06441
- WO-A-90/00389
- GB-A- 1 192 479
- GB-A- 2 085 729
- DATABASE WPI Week 9335 Derwent Publications Ltd., London, GB; AN 93-278189 XP002007530 & JP-A-05 194 196 (EISAI CO LTD) , 3.August 1993
- DATABASE WPI Week 8908 Derwent Publications Ltd., London, GB; AN 89-057692 XP002007531 & JP-A-01 009 931 (DAIICHI SEIYAKU KK) , 13.Januar 1989

## Beschreibung

Die vorliegende Erfindung betrifft ein Verfahren zur Herstellung einer liposomalen, in Wasser dispergierbaren, oral zu verabreichenden, festen, trockenen therapeutischen Formulierung.

Es ist eine Tatsache, dass biologisch aktive, lipophile Wirkstoffe, welche oral verabreicht werden, schlecht im gastrointestinalen Trakt absorbierbar sind und eine entsprechend geringe und variable Bioverfügbarkeit zeigen.

Lipophile Wirkstoffe, welche oral verabreicht werden, werden meistens als konzentrierte Oellösungen, Emulsionen oder Mikroemulsionen formuliert.

Solche Formulierungen haben den Nachteil einer ungenügenden Dispersion in der gastrointestinalen Flüssigkeit.

Dieser Nachteil wird mit relativ grossen Mengen an Tensiden kompensiert.

Die Bioverfügbarkeit solcher Formulierungen ist zudem stark abhängig von im gastrointestinalen Trakt vorhandener Konzentration an Gallensäure.

Ein bekanntes Beispiel dieser Problematik ist die Bereitstellung einer Formulierung von Cyclosporin A.

Siehe beispielsweise DE OS 39 30 928 und EP 0 589 843 A1.

Es ist ein Ziel der vorliegenden Erfindung, ein einfaches, billiges und industriell realisierbares Verfahren zur Herstellung einer oralen Formulierung von biologisch aktiven, lipophilen Wirkstoffen zur Verfügung zu stellen.

Es soll eine feste und stabile Formulierung hergestellt werden, welche in Wasser eine hochdisperse Dosierung des genannten lipophilen Wirkstoffes ermöglicht.

Diese Formulierung soll verbesserte Parameter der Bioverfügbarkeit haben.

Diese Formulierung soll in der Form eines Pulvers oder Granulates zur Verfügung gestellt werden, verbunden mit optimalen Dosierungseigenschaften.

Es ist gefunden worden, dass die obigen Ziele erreicht werden können, wenn die biologisch aktiven, lipophilen Wirkstoffe in liposomalen Teilchen eingebettet werden.

Liposomen und Verfahren zu deren Herstellung werden beispielsweise ganz allgemein beschrieben in "Liposomes, a practical approach", RRC New, IRL Press, Oxford University Press, Walton Street, Oxford 0X2 6DP, (1990).

In der US PS 4 830 858 wird eine Methode beschrieben, bei der zwecks Stabilisierung preliposomale Komponenten sprühgetrocknet werden.

In Beispiel 1 von EP 0 170 642 A2 wird die Herstellung einer Formulierung für die Instillation beschrieben.

Dabei wird aus dem Lipid Dipalmitoylphosphatidyl-cholin, dem Stabilisator Cholesterol und dem Wirkstoff Budesonid-21-palmitat in Gegenwart von Chloroform eine Emulsion gebildet.

Danach wird das Lösungsmittel entfernt, und eine wässrige NaCl-Lösung wird hinzugegeben.

Die Liposomen werden durch Schütteln oder durch Beschallung gebildet.

In Beispiel 2 von EP 0 170 642 A2 wird die Herstellung einer Formulierung für die Inhalation beschrieben, wobei Lactose als Hilfsstoff für die Lyophilisation verwendet wird.

Die liposomale Suspension wird lyophilisiert.

Das trockene Produkt muss für die angegebene Verwendung auf eine Teilchengrösse von etwa 2 Mikrometer mikronisiert werden.

Bei der Anwendung dieser Produkte ist keine homogene Verteilung der wasserunlöslichen Wirkstoffe in der Membrankomponente gewährleistet.

S. Mihailova et. al., Congr. Int. Technol. Pharm., 6th Assoc. Pharm. Galenique Ind., Chatenay Malabry (FR), Seiten 346 bis 353 (1992), beschreibt die Herstellung einer liposomalen Suspension von Cyclophosphamid. Dieses Verfahren beinhaltet einen Filtrationsprozess, wobei Wirkstoffanteile verloren gehen. Das Produkt liegt in nicht getrockneter Form vor.

Das erfindungsgemässe Verfahren zur Herstellung einer liposomalen, in Wasser dispergierbaren, oral zu verabreichenden, festen, trockenen therapeutischen Formulierung, wobei man
- in einem ersten Schritt wenigstens einen biologisch aktiven, lipophilen Wirkstoff, wenigstens ein Lipid, welches Vesikel bildet, und wenigstens eine Komponente, welche die Vesikelstruktur stabilisiert und/oder als Lösungsvermittler dient, in wenigstens einem organischen Lösungsmittel löst, dann
- in einem zweiten Schritt aus der erhaltenen homogenen Lösung das (die) organische(n) Lösungsmittel entfernt, dann
- in einem dritten Schritt das so erhaltene homogene Gemisch hydratisiert, dann
- in einem vierten Schritt die so erhaltene Suspension so lange mechanisch behandelt, bis der genannte Wirkstoff vollständig und homogen verteilt in den entstandenen liposomalen Teilchen enthalten ist,
ist dadurch gekennzeichnet, dass man
- in einem fünften Schritt noch wenigstens ein Trägermaterial hinzugibt, und dann das Wasser mittels Sprühtrocknung oder im Granulationsverfahren entfernt und den so erhaltenen Festkörper, welcher die Form eines Pulvers oder Granulates hat, gewinnt.

Die erfindungsgemässe liposomale, in Wasser dispergierbare, oral zu verabreichende, feste, trockene, therapeutische Formulierung, ist dadurch gekennzeichnet, dass sie wenigstens einen biologisch aktiven, lipophilen Wirkstoff homogen verteilt in liposomalen Teilchen enthält, wobei diese liposomalen Teilchen aufgebaut sind aus wenigstens einem Lipid, welches Vesikel bildet, wenigstens einer Komponente, welche die Vesikelstruktur stabilisiert und/oder als Lösungsvermittler dient, und wenigstens einem Trägermaterial, und dass diese Formulierung die Form eines Pulvers oder Granulates hat.

Nachfolgend werden Vorteile, wesentliche Akpekte der vorliegenden Erfindung und bevorzugte Ausführungsformen der vorliegenden Erfindung näher beschrieben.

Dabei werden Ausführungsformen, welche in den abhängigen Ansprüchen definiert sind, normalerweise nicht wiederholt.

Liposomen bestehen bekanntlich aus einer lipophilen Membran, welche einen Wasserkern umhüllt.

In der vorliegenden Erfindung ist es wesentlich, dass der biologisch aktive, lipophile Wirkstoff von der lipophilen Membran aufgenommen wird und nicht in fester Form im Wasserkern enthalten ist.

Das genannte homogene Gemisch kann die Form eines Filmes oder eines Pulvers haben. Bevorzugt wird ein Pulver, welches mittels Sprühtrocknung hergestellt wird. Mit diesem Pulver wird eine optimale Benetzung mit Wasser im nachfolgenden Schritt erreicht.

Speziell gute Resultate werden erhalten, wenn bestimmte Mol- und Gewichtsverhältnisse, wie sie in den abhängigen Ansprüchen definiert sind, zwischen den einzelnen Komponenten gewählt werden.

Eine homogene Verteilung der genannten Wirkstoffe wird insbesondere erreicht, wenn die liposomalen Teilchen hauptsächlich eine unilamellare Struktur aufweisen und der Bereich der mittleren Durchmesser der liposomalen Teilchen möglichst eng ist.

Dazu ist eine reproduzierbare mechanische Behandlung notwendig. Speziell geeignet sind Hochdruck-Homogenisatoren.

Geeignet ist ein Druck im Bereich von 400 bar bis 1000 bar.

Kleinere Mengen können auch mit Ultraschall behandelt werden.

Die anschliessende Entfernung des Wassers mittels Sprühtrocknung oder Granulationsverfahren erlaubt die Herstellung eines fliessfähigen, festen, stabilen und trockenen Produktes.

Dieses Produkt kann in Sachets oder Kapseln, insbesondere Hartgelatinekapseln, abgefüllt werden und einfach dosiert werden.

Das erfindungsgemässe Verfahren kann unter einer Inertgasatmosphäre, beispielsweise Stickstoff oder Argon, durchgeführt werden.

Synthetische Lipide, welche in der vorliegenden Erfindung verwendet werden können, sind beispielsweise bei der Firma Sygena Ltd. in CH-4410 Liestal erhältlich.

Das erfindungsgemässe Verfahren enthält keine Schritte, welche eine Ausführung dieses Verfahrens im grosstechnischen Massstab verhindern könnten.

Die nachfolgenden Beispiele illustrieren die vorliegende Erfindung.

### Beispiel 1

5 g Cyclosporin A wurden in 300 ml absolutem Ethanol bei Raumtemperatur unter Rühren gelöst.

Dann wurden gleichzeitig 50 g Sojalecithin und 8 g Cholesterol hinzugegeben und unter Temperaturerhöhung bis 50°C ebenfalls gelöst.

Man erhielt eine klare orangefarbige Lösung, welche auf Raumtemperatur abgekühlt wurde. Durch diese Lösung wurde Argon hindurchgeblasen.

Diese Lösung wurde in einer Büchi 190 Sprühtrocknungsapparatur sprühgetrocknet, und das erhaltene Pulver wurde direkt in 300 ml einer gerührten Pufferlösung gegeben.

Diese Pufferlösung war 130 millimolar an Natriumchlorid und 10 millimolar an Natriumdihydrogenphosphat und wurde mittels Hinzugabe von 20%-iger Natriumhydroxidlösung auf einen pH-Wert von 7,4 gebracht.

Die resultierende milchige Suspension wurde zuerst während 1 Minute mit externer Eisbadkühlung ein einem Ystral X 10/20 Homogenisator bei 8000 Umdrehungen pro Minute behandelt. Für die Spitze wurde das Modell 21/G verwendet.

Durch das erhaltene Produkt wurde Argon hindurchgeblasen. Dieses Produkt wurde 3 x durch einen M-110 T Laboratory Microfluidizer bei einem Druck von 400 bis 500 bar geführt.

In 33 g des so erhaltenen Produktes wurden unter Rühren bei Raumtemperatur 18 g Maltodextrin gelöst.

Das resultierende Produkt wurde in einer Büchi 190 Sprühtrocknungsapparatur sprühgetrocknet.

28 g des erhaltenen leicht crèmefarbigen Pulvers wurden mit 56 mg Natriumdodecylsulfat vermischt.

Dieses Gemisch wurde gemäss der HPLC-Methode, welche in USP 23 beschrieben ist, analysiert.

Es wurde ein Gehalt von 5,15 % an Cyclosporin A ermittelt (theoretischer Wert: 4,78 %).

Bei den beiden oben erwähnten Sprühtrocknungen wurden folgende Parameter verwendet:

| | 1.Sprühtrocknung | 2.Sprühtrocknung |
|---|---|---|
| Einlasstemperatur | 54°C | 141°C |
| Auslasstemperatur | 45°C | 93°C |
| Sprühfluss (spray rate) | 4,8 ml/min | 5,4 ml/min |
| Luftstrom (Druckluft) | 750 l/Stunde | 750 l/Stunde |
| | | |
| Druck der Druckluft | 5 bar | 5 bar |
| Durchmesser der Düse | 0,7 mm | 0,7 mm. |

### Beispiel 2

Analog Beispiel 1 bis und mit der Behandlung im Microfluidizer.

44 g Maltodextrin wurden auf einem Wirbelbett Typ Aeromatic Laboratory Unit Serie 1 vorgelegt, und darauf wurde das erhaltene Produkt aufgesprüht.

Für dieses Aufsprühen wurden folgende Parameter verwendet:

| | |
|---|---|
| Einlasstemperatur | 68°C bis 75°C |
| Auslasstemperatur | 47°C bis 51°C |
| Sprühfluss (spray rate) | 0,8 ml/min |
| Luftstrom (Druckluft) | 130 m ³ /Stunde |
| Druck der Druckluft | 1,4 bar |
| Durchmesser der Düse | 1,1 mm |
| Ablassdruck im | 2 bar. |
| Wirbelbett | |

Man erhielt 80 g eines crèmefarbigen Granulates.

Analog Beispiel 1 wurde ein Gehalt von 3,12 % an Cyclosporin A ermittelt.

## Patentansprüche

1. Verfahren zur Herstellung einer liposomalen, in Wasser dispergierbaren, oral zu verabreichenden, festen, trockenen therapeutischen Formulierung, wobei man
- in einem ersten Schritt wenigstens einen biologisch aktiven, lipophilen Wirkstoff, wenigstens ein Lipid, welches Vesikel bildet, und wenigstens eine Komponente, welche die Vesikelstruktur stabilisiert und/oder als Lösungsvermittler dient, in wenigstens einem organischen Lösungsmittel löst, dann
- in einem zweiten Schritt aus der erhaltenen homogenen Lösung das (die) organische(n) Lösungsmittel entfernt, dann
- in einem dritten Schritt das so erhaltene homogene Gemisch hydratisiert, dann
- in einem vierten Schritt die so erhaltene Suspension so lange mechanisch behandelt, bis der genannte Wirkstoff vollständig und homogen verteilt in den entstandenen liposomalen Teilchen enthalten ist,
**dadurch gekennzeichnet, dass** man
- in einem fünften Schritt noch wenigstens ein Trägermaterial hinzugibt, und dann das Wasser mittels Sprühtrocknung oder im Granulationsverfahren entfernt und den so erhaltenen Festkörper, welcher die Form eines Pulvers oder Granulates hat, gewinnt.

2. Verfahren nach Anspruch 1, **dadurch gekennzeichnet, dass** der Wirkstoff ausgewählt ist aus der Gruppe, bestehend aus Peptiden, vorzugsweise ein Cyclosporin, beispielsweise Ciclosporin, auch Cyclosporin A genannt, Muramyl-Peptide, entzündungshemmenden steroidalen Wirkstoffen, wie Prednison, Dexamethason, entzündungshemmenden, nicht-steroidalen Wirkstoffen, wie Indomethazin, Zytostatika, wie Doxorubicin, Antimykotika, wie Myconazol, Amphotericin B.

3. Verfahren nach einem der Ansprüche 1 bis 2, **dadurch gekennzeichnet, dass** das Lipid synthetischer oder natürlicher Herkunft ist, insbesondere ein Phospholipid, wie Phosphatidylcholin, Phosphatidylethanolamin, Phosphatidylglycerole.

4. Verfahren nach einem der Ansprüche 1 bis 3, **dadurch gekennzeichnet, dass** die Komponente, welche die Vesikelstruktur stabilisiert und/oder als Lösungsvermittler dient, ein Sterol ist, wie Cholesterol oder Stigmasterol.

5. Verfahren nach einem der Ansprüche 1 bis 4, **dadurch gekennzeichnet, dass** das organische Lösungsmittel ein Alkohol, wie Ethanol oder Isopropanol, oder Chloroform ist.

6. Verfahren nach einem der Ansprüche 1 bis 5, **dadurch gekennzeichnet, dass** man im zweiten Schritt das (die) organische(n) Lösungsmittel mittels Sprühtrocknung derart entfernt, dass ein mit Wasser benetzbares Pulver entsteht.

7. Verfahren nach einem der Ansprüche 1 bis 6, **dadurch gekennzeichnet, dass** im dritten Schritt die Hydratisierung in einer Pufferlösung erfolgt, vorzugsweise mit einem pH-Wert von 5 bis 8.

8. Verfahren nach einem der Ansprüche 1 bis 7, **dadurch gekennzeichnet, dass** im vierten Schritt die mechanische Behandlung mittels Homogenisierung oder Ultraschallbehandlung erfolgt, insbesondere bei einer Temperatur im Bereich von 5°C bis 60°C.

9. Verfahren nach einem der Ansprüche 1 bis 8, **dadurch gekennzeichnet, dass** das Trägermaterial ein Monosaccharid, wie Glukose, ein Disaccharid, wie Lactose, oder Polysaccharide, oder Gemische davon, wie Maltodextrin, ist.

10. Verfahren nach einem der Ansprüche 1 bis 9, **dadurch gekennzeichnet, dass** das Molverhältnis zwischen Wirkstoff, beispielsweise Cyclosporin A, und Lipid, beispielsweise Lecithin, derart gewählt ist, dass die Aufnahmekapazität an Wirkstoff in den Liposomen nicht überschritten wird.

11. Verfahren nach einem der Ansprüche 1 bis 10, **dadurch gekennzeichnet, dass** das Gewichtsverhältnis zwischen Wirkstoff, beispielsweise Cyclosporin A, und der Komponente, welche die Vesikelstruktur stabilisiert und/oder als Lösungsvermittler dient, beispielsweise Cholesterol, von 1 zu 1 bis 1 zu 2 beträgt.

12. Verfahren nach einem der Ansprüche 1 bis 11, **dadurch gekennzeichnet, dass** das Gewichtsverhältnis zwischen Lipid und der Komponente, welche die Vesikelstruktur stabilisiert und/oder als Lösungsvermittler dient, von 5 zu 1 bis 10 zu 1 beträgt.

13. Verfahren nach einem der Ansprüche 1 bis 12, **dadurch gekennzeichnet, dass** das erhaltene Pulver oder Granulat von 3 bis 10 Gew.-%, insbesondere von 5 bis 7 Gew.-%, an Wirkstoff enthält.

14. Verfahren nach einem der Ansprüche 1 bis 13, **dadurch gekennzeichnet, dass** das erhaltene Pulver oder Granulat von 20 bis 80 Gew.-%, insbesondere von 30 bis 60 Gew.-%, an Trägermaterial enthält.

15. Verfahren nach einem der Ansprüche 1 bis 14, **dadurch gekennzeichnet, dass** man das erhaltene Pulver oder Granulat mit wenigstens einem pulverförmigen Netzmittel vermischt, beispielsweise Natriumdodecylsulfat, Natriumcetylstearylsulfat, insbesondere in einem Gewichtsverhältnis von 1 zu 1000 bis 1 zu 500.

## Claims

1. A process for the preparation of a liposomal, in water dispersable, orally administerable, solid, dry therapeutic formulation, whereby
- in a first step at least one biologically active, lipophilic component, at least one lipid, which forms vesicles, and at least one component, which stabilizes the vesicle structure and/or serves as solubilizer, are dissolved in at least one organic solvent, then
- in a second step from the obtained homogeneous solution the organic solvent(s) is (are) removed, then
- in a third step the so obtained homogeneous mixture is hydrated, then
- in a fourth step the so obtained suspension is treated mechanically during such a long time, until said active component is contained completely and homogeneously distributed in the formed liposomal particles,
**characterized in that**
- in a fifth step at least one carrier material is added, and then the water is removed by means of spray drying or by means of a granulating process and the so obtained solid, which has the form of a powder or of granules, is obtained.

2. The process according to claim 1, **characterized in that** the active component is selected from the group consisting of peptides, preferably a cyclosporin, for example ciclosporin, also named cyclosporin A, muramyl-peptides, anti-inflammatory steroidal active components, such as prednisone, dexamethasone, anti-inflammatory, non-steroidal active components, such as indomethacin, antitumor agents, such as doxorubicin, antimycotics, such as myconazol, amphotericin B.

3. The process according to one of claims 1 to 2, **characterized in that** the lipid is of synthetic or of natural origin, especially a phospholipid, such as phosphatidylcholine, phosphatidylethanolamine, phosphatidylglycerols.

4. The process according to one of claims 1 to 3, **characterized in that** the component, which stabilizes the vesicle structure and/or serves as solubilizer, is a sterol, such as cholesterol or stigmasterol.

5. The process according to one of claims 1 to 4, **characterized in that** the organic solvent is an alcohol, such as ethanol or isopropanol, or chloroform.

6. The process according to one of claims 1 to 5, **characterized in that** in the second step the organic solvent(s) is(are) removed by means of spray drying resulting in a with water wettable powder.

7. The process according to one of claims 1 to 6, **characterized in that** in the third step the hydration is realized in a buffer solution, preferably with a pH-value from 5 to 8.

8. The process according to one of claims 1 to 7, **characterized in that** in the fourth step the mechanical treatment is realized by means of homogenization or by means of treatment with ultrasonics, especially at a temperature in the range from 5°C to 60°C.

9. The process according to one of claims 1 to 8, **characterized in that** the carrier material is a monosaccharide, such as glucose, a disaccharide, such as lactose, or polysaccharides, or mixtures thereof, such as maltodextrin.

10. The process according to one of claims 1 to 9, **characterized in that** the molar ratio between active component, for example cyclosporin A, and lipid, for example lecithin, is choosen such, that the absorption capacity of active component in the liposomes is not exceeded.

11. The process according to one of claims 1 to 10, **characterized in that** the weight ratio between active component, for example cyclosporin A, and the component, which stabilizes the vesicle structure and/or serves as solubilizer, for example cholesterol, is from 1 to 1 up to 1 to 2.

12. The process according to one of claims 1 to 11, **characterized in that** the weight ratio between lipid and the component, which stabilizes the vesicle structure and/or serves as solubilizer, is from 5 to 1 up to 10 to 1.

13. The process according to one of claims 1 to 12, **characterized in that** the obtained powder or granules contain(s) from 3 to 10 % by weight, especially from 5 to 7 % by weight, of active component.

14. The process according to one of claims 1 to 13, **characterized in that** the obtained powder or granules contain(s) from 20 to 80 % by weight, especially from 30 to 60 % by weight, of carrier material.

15. The process according to one of claims 1 to 14, **characterized in that** the obtained powder or granules is (are) mixed with at least one powdery wetting agent, for example sodium dodecyl sulfate, sodium cetyl stearyl sulfate, especially in a weight ratio from 1 to 1000 up to 1 to 500.

## Revendications

1. Procédé de préparation d'une formulation thérapeutique, liposomique, solide, sèche, dispersible dans l'eau, pour administration orale, où :
- dans une première étape, on dissout au moins un agent biologiquement actif lipophile, au moins un lipide, qui forme des vésicules, et au moins un composant qui stabilise la structure des vésicules et/ou sert de solubilisant, dans au moins un solvant organique ; puis
- dans une deuxième étape, on élimine le ou les solvants organiques de la solution homogène obtenue, puis
- dans une troisième étape, on hydrate le mélange homogène ainsi obtenu, puis
- dans une quatrième étape, on traite mécaniquement la suspension ainsi obtenue jusqu'à ce que l'agent actif cité soit dispersé complètement et de manière homogène dans les particules liposomiques formées,
**caractérisé en ce que** :
- dans une cinquième étape, on ajoute encore au moins un matériau support et puis on élimine l'eau par séchage par pulvérisation ou par un procédé de granulation et on recueille le corps solide ainsi obtenu, qui a la forme d'une poudre ou d'un granulé.

2. Procédé suivant la revendication 1,
**caractérisé en ce que** l'agent actif est choisi parmi le groupe consistant en des peptides, de préférence une cyclosporine, par exemple la ciclosporine également dite cyclosporine A, les peptides muramyle, des agents anti-inflammatoires stéroïdiens, comme la prednisone, la dexaméthasone, des agents anti-inflammatoires non stéroïdiens, comme l'indométhazine, un cytostatique comme la doxorubicine, un antimycotique comme le myconazole, l'amphotéricine B.

3. Procédé suivant l'une quelconque des revendications 1 et 2, **caractérisé en ce que** le lipide est d'origine synthétique ou naturelle, en particulier un phospholipide comme la phosphatidylcholine, la phosphatidyléthanolamine, le phosphatidylglycérol.

4. Procédé suivant l'une quelconque des revendications 1 à 3, **caractérisé en ce que** le composant qui stabilise la structure des vésicules et/ou sert de solubilisant, est un stérol comme le cholestérol ou le stigmastérol.

5. Procédé suivant l'une quelconque des revendications 1 à 4, **caractérisé en ce que** le solvant organique est un alcool comme l'éthanol ou l'isopropanol, ou le chloroforme.

6. Procédé suivant l'une quelconque des revendications 1 à 5, **caractérisé en ce que** l'on sépare le ou les solvants organiques dans la deuxième étape par un séchage par pulvérisation de sorte qu'il se forme une poudre mouillable par l'eau.

7. Procédé suivant l'une quelconque des revendications 1 à 6, **caractérisé en ce que** dans la troisième étape, l'hydratation est réalisée dans une solution tampon, de préférence à un pH allant de 5 à 8.

8. Procédé suivant l'une quelconque des revendications 1 à 7, **caractérisé en ce que** dans la quatrième étape, le traitement mécanique est réalisé à l'aide d'une homogénéisation ou d'un traitement aux ultra-sons, en particulier à une température située dans l'intervalle allant de 5°C à 60°C.

9. Procédé suivant l'une quelconque des revendications 1 à 8, **caractérisé en ce que** le matériau support est un monosaccharide comme le glucose, un disaccharide comme le lactose, ou un polysaccharide ou leurs mélanges, comme la maltodextrine.

10. Procédé suivant l'une quelconque des revendications 1 à 9, **caractérisé en ce que** le rapport molaire entre l'agent actif, par exemple la cyclosporine A, et le lipide, par exemple la lécithine, est choisi de sorte que la capacité d'absorption de l'agent actif dans les liposomes ne soit pas dépassée.

11. Procédé suivant l'une quelconque des revendications 1 à 10, **caractérisé en ce que** le rapport pondéral entre l'agent actif, par exemple la cyclosporine A, et le composant qui stabilise la structure des vésicules et/ou sert de solubilisant, par exemple le cholestérol, se situe dans l'intervalle allant de 1:1 à 1:2.

12. Procédé suivant l'une quelconque des revendications 1 à 11, **caractérisé en ce que** le rapport pondéral entre le lipide et le composant qui stabilise la structure des vésicules et/ou sert de stabilisant, se situe dans l'intervalle allant de 5:1 à 10:1.

13. Procédé suivant l'une quelconque des revendications 1 à 12, **caractérisé en ce que** la poudre ou le granulé obtenu contient de 3 à 10% en poids, en particulier de 5 à 7% en poids, d'agent actif.

14. Procédé suivant l'une quelconque des revendications 1 à 13, **caractérisé en ce que** la poudre ou le granulé obtenu contient de 20 à 80% en poids, en particulier de 30 à 60% en poids, de matériau support.

15. Procédé suivant l'une quelconque des revendications 1 à 14, **caractérisé en ce que** l'on mélange la poudre ou le granulé obtenu avec au moins un agent mouillant en poudre, par exemple du dodécylsulfate de sodium, du cétylstéarylsulfate de sodium, en particulier en un rapport pondéral situé dans l'intervalle allant de 1:1000 à 1:500.
